# EUROPÄISCHE PATENTSCHRIFT

(11) **EP 1 741 458 B1**
(45) Veröffentlichungstag und Bekanntmachung des Hinweises auf die Patenterteilung: **25.01.2012**
(21) Anmeldenummer: 06012357.7
(22) Anmeldetag: 16.06.2006
(51) Int. Cl.: A61M 1/16, A61M 1/36, A61M 1/10

(54) **Vorrichtung zur Behandlung von Blut in einem extrakorporalen Blutkreislauf**
Device for treating blood in an extracorporeal blood circuit
Dispositif de traitement de sang dans un circuit sanguin extracorporel

(30) Priorität: 06.07.2005 DE 102005031582
(43) Veröffentlichungstag der Anmeldung: 10.01.2007
(73) Patentinhaber: Maquet Cardiopulmonary AG, 72145 Hirrlingen (DE)
(72) Erfinder: Blickle, Rainer, 72475 Bitz (DE); Haag, Ulrich, 72406 Bisingen (DE); Küper, Enno-Utz, 72144 Dusslingen (DE)
(74) Vertreter: Kohler Schmid Möbus

(56) Entgegenhaltungen:
- EP-A- 1 382 358
- EP-A- 1 464 350
- WO-A-93/01846
- DE-A1- 3 733 542
- US-A- 4 396 584
- US-A- 4 424 190
- US-A- 5 043 140
- US-A- 5 411 706
- US-A- 5 429 184
- US-A- 5 770 149
- US-A- 5 823 987
- US-A1- 2004 009 097
- US-B1- 6 241 945

## Beschreibung

Die Erfindung betrifft eine Vorrichtung zur Behandlung von Blut in einem extrakorporalen Blutkreislauf, die mindestens einen Oxygenator, einen Wärmetauscher und ein Blutfilter aufweist.

Eine derartige Vorrichtung ist durch die EP 1 464 350 A1 bekannt geworden.

Die bekannte Vorrichtung weist in einem Gehäuse eine Blasenfalle, eine Blutpumpe, einen Wärmetauscher, einen Oxygenator und ein arterielles Blutfilter auf. Die einzelnen Vorrichtungskomponenten sind eng aneinander gekoppelt, sodass gegenüber Komponenten, die über Leitungssysteme miteinander fluidtechnisch verbunden sind, kürzere Leitungswege erreicht werden. Die Leitungswege selbst stellen aber auch Verengungen dar, die das aufzubereitende Blut belasten, sodass diese Querschnittsverengungen einer schonenden Blutaufbereitung entgegenwirken.

Ferner ist aus DE 37 33 542 A1 ein Blutoxygenator bekannt, der aus Komponenten zusammengesetzt ist, die aneinandergereiht werden. Das Blutfilter wird bei dieser Vorrichtung über einen Verteiler oder radial über den Gehäusemantel mit den übrigen Vorrichtungskomponenten verbunden. Dadurch verlängern sich die Blutströmungswege in den bekannten Blutoxygenator.

Eine Apparatur und ein Verfahren zur Förderung und Oxygenierung von Blut ist aus US 2004/009097 A1 bekannt. Die in einem Gehäuse untergebrachten Komponenten weisen unterschiedlichste Strömungsübergangsquerschnitte auf, und Oxygenator und Wärmetauscher sind nebeneinander angeordnet.

Aus US 5,043,140 ist eine Vorrichtung zur Blutoxygenierung bekannt, bei der zwischen dem Oxygenator/Wärmetauscher und einem Blutfilter ein Entschäumer angeordnet ist. Ein Röhrenwärmetauscher ist im Oxygenator vorgesehen. Übergangsströmungsquerschnitte der in einem Gehäuse zusammengefügten Komponenten werden in der Druckschrift nicht beschrieben.

Weiterhin ist aus US 6,241,945 B1 eine modulartig aufgebaute Vorrichtung zur Entgasung, Oxygenierung und Filterung von Blut bekannt. Einzelne Funktionsmodule werden aneinandergereiht. Mehrere Funktionen werden nicht in einem Funktionsmodul zusammengefasst, und in welcher Art und Weise ein zusätzlicher Wärmetauscher mit der Vorrichtung zu verbinden ist, ist nicht beschrieben.

US 5,411,706 offenbart ein System aus Oxygenator und Zentrifugalpumpe, wobei eine interne Rezirkulation des zu behandelnden Blutes vorgesehen ist. Optional kann ein Wärmetauscher und/oder ein Reservoir mit Filter mit dem System verbunden werden. Der Wärmetauscher ist nicht in den Oxygenator integriert, sodass die Länge der Strömungswege des zu behandelnden Blutes von der Länge der vorgesehenen Leitungen abhängen.

Schließlich ist aus US 5,770,149 eine Vorrichtung zur extrakorporalen Behandlung von Blut bekannt geworden, bei der in einem Gehäuse die einzelnen Komponenten aneinandergereiht sind. Das zu behandelnde Blut wird im Gehäuse über eine Pumpe einem wendelartig aufgebauten Wärmetauscher zugeführt. An den Wärmetauscher schließt sich ein Oxygenator an, der im Gehäuse mit einem Blutfilter verbunden ist. Bei dieser Vorrichtung sind die Komponenten Wärmetauscher, Oxygenator getrennt voneinander in einem Gehäuse integriert. Die Größe der Strömungsquerschnitte von Komponente zu Komponente im Gehäuse sind für diese Vorrichtung weder angegeben, noch werden Empfehlungen dafür gegeben. Die Aneinanderreihung von Wärmetauscher und Oxygenator führt zu verlängerten Strömungswegen des Blutes in der Vorrichtung.

Aufgabe der Erfindung ist es, eine Vorrichtung zur Behandlung von Fluid in einem extrakorporalen Blutkreislauf derart weiterzubilden, dass sie einerseits für erweiterte Indikationen sicher und schnell einsetzbar ist und andererseits das extrakorporal zirkulierende Blut bestmöglich schont.

Erfindungsgemäß wird diese Aufgabe durch die Merkmale des Patentanspruchs 1 gelöst.

Die Vorrichtungskomponenten Oxygenator, Wärmetauscher und Blutfilter bilden unter Verzicht auf Verbindungsleitungen oder -kanäle ein Kompaktmodul in einem Gehäuse.

Die erfindungsgemäße Vorrichtung hat damit den wesentlichen Vorteil, dass Verengungen innerhalb der Vorrichtung bei der Behandlung des Blutes minimiert werden, das aufzubereitende Blut kann über große Strömungsquerschnitte zwischen den Vorrichtungskomponenten strömen und erfährt dadurch eine erheblich geringere Scherbelastung als in aus dem Stand der Technik bekannten Strömungswegen, die Verbindungsleitungen oder direkt verbundene Kanäle zwischen zwei Vorrichtungskomponenten betreffen. Durch den Verzicht auf die die Blutbahn verengenden Verbindungsleitungen oder -kanäle wird der Druckabfall über die Vorrichtung minimiert, daraus folgt eine Reduzierung der notwendigen Pumpenleistung mit entsprechend verringerter Blutschädigung. Darüber hinaus wird durch die kompakte Bauweise das sich im extrakorporalen Kreislauf befindliche Blutvolumen sowie das für das Priming, d. h. für die Inbetriebnahme des Kompaktmoduls benötigte Flüssigkeitsvolumen reduziert. Die Verweildauer des extrakorporal strömenden Blutes wird durch die erfindungsgemäße Ausbildung der Ein- und Auslässe der Vorrichtungskomponenten erheblich verkürzt, weil große Ein- und Auslassquerschnitte der Vorrichtungskomponenten direkt zusammengekoppelt sind und dadurch Druckverluste innerhalb der erfindungsgemäßen Vorrichtung minimiert werden. Die Blutdurchflussquerschnitte sind größer und/oder gleich 120 mm² ausgestaltet und dies immer im Übergangsbereich der Vorrichtungskomponenten zueinander, so wird das extrakorporal zirkulierende Blut weiter geschont und auf das Blut wirkende Scherkräfte bzw. der auf das Blut strömungstechnisch wirkende Druck wird minimiert. Somit kann die erfindungsgemäße Vorrichtung mit einer geringeren Pumpleistung betrieben werden, die Blut schädigenden Einflüssen entgegenwirkt.

Der Wärmetauscher ist in den Oxygenator integriert und der Oxygenator und der Wärmetauscher bilden ein untrennbares Bauteil. Dies hat den Vorteil, dass eine sehr wirkungsvolle Wärmetauschleistung erreicht werden kann, indem Wärmetauscherfasern in den Oxygenator integriert sind. Die Wärmetauscherfasern können beispielsweise elektrostatische Aufladungen noch wirkungsvoller ableiten und verbessern die Leistung des Wärmetauschers. In der erfindungsgemäßen Ausbildung bilden der Wärmetauscher und der Oxygenator ein untrennbares Bauteil und die Funktionskomponenten von Wärmetauscher und Oxygenator bilden eine einzige Einheit. Dies erlaubt eine sehr kompakte Bauweise und verbessert den Wirkungsgrad im Zusammenwirken von Wärmetauscher und Oxygenator.

In weiterer Ausgestaltung der Erfindung weisen die Öffnungen unterschiedlich große Öffnungsquerschnitte auf. Dies hat den Vorteil, dass große Öffnungen im oberen Teil der Baueinheit verbessert zur Gasblasenabführung aus dem Blut zusätzlich benützt werden können. Je nach Bedarf und konstruktiver Ausgestaltung des Bauteils können die Öffnungen an verschiedensten Stellen des Bauteils angebracht werden und somit werden auch Bauteiloptimierungen unterstützt, die bei einem einzigen Einlass und bei einem einzigen Auslass nicht möglich sind.

Besondere Vorteile ergeben sich dann, wenn vier Öffnungen über den Umfang der Baueinheit bzw. des Bauteils vorgesehen sind. Über vier Öffnungen können nicht nur Leitungssysteme bevorzugt schonend und schnell das aufzubereitende Blut in das Bauteil Oxygenator/Wärmetauscher transportieren, sondern die Öffnungen erlauben auch eine direkte Verbindung mit angrenzenden Vorrichtungskomponenten.

An die Baueinheit kann am Einlass eine Pumpe, insbesondere eine Zentrifugalpumpe vorgesehen werden, über die das aufzubereitende Blut in den Oxygenator/Wärmetauscher einströmt. Der Auslass der Pumpe ist auf den Einlass an der Baueinheit abgestimmt, sodass ohne Druckverlust das aufzubereitende Blut von der Pumpe in das Bauteil Oxygenator/Wärmetauscher einströmen kann.

Besondere Vorteile ergeben sich dann, wenn Öffnungen jeweils am oberen Teil der Vorrichtung und am unteren Teil der Vorrichtung vorhanden sind. Die Öffnungen am oberen Teil der Vorrichtung ermöglichen eine einfache und effektive blasenfreie Befüllung der Zentrifugalpumpe, die Öffnungen am unteren Teil der Vorrichtung ermöglichen eine einfache und vollständige Entleerung des Pumpenraums am Ende des extrakorporalen Blutkreislaufs, wenn das extrakorporal befindliche Blut möglichst vollständig in den Patienten zurückgegeben werden soll.

Ist die Zentrifugalpumpe über einen Anschluss an die Baueinheit oder das Bauteil (Oxygenator/Wärmetauscher) angekoppelt und sind die Blut führenden Verbindungsöffnungen oben und unten angeordnet, unabhängig von deren Größen der Öffnungsquerschnitte, so ist eine bevorzugte, einfache und effektive Entgasung bzw. Entleerung des Kompaktmoduls möglich.

Um kurze Strömungswege zu gewährleisten, ist die Pumpe an die Baueinheit unmittelbar ankoppelbar. Verlängerte Leitungswege werden durch diese Maßnahme unterbunden und die gesamte Vorrichtung lässt sich durch diese Maßnahme kompakt aufbauen. Je nach Bedarf lässt sich an die Baueinheit eine Pumpe ankoppeln, sodass eine derartige Vorrichtung an einen erweiterten Einsatzbereich bzw. erweiterte Anforderungen einfachst anpassbar ist.

Im Bedarfsfall ist es auch möglich, dass an die Baueinheit eine Blasenfalle oder ein Blutreservoir angekoppelt wird, das vor der Pumpe vorgesehen und mit der Pumpe fluidtechnisch verbunden ist.

Somit ist die erfindungsgemäße Vorrichtung für verschiedenste Einsätze zur Aufrechterhaltung eines extrakorporalen Blutkreislaufs einsetzbar und ohne konstruktive Umgestaltungen aufwändigster Art kann die Vorrichtung verändert werden. Alle benötigten Vorrichtungskomponenten können in einem Gehäuse vereinigt werden und an die Pumpe ist beispielsweise ein Antrieb ankoppelbar, der mit dem aufzubereitenden Blut nicht in Berührung kommt.

Nachfolgend ist die erfindungsgemäße Vorrichtung in einem Ausführungsbeispiel beschrieben, in dem eine Ausführungsmöglichkeit unter mehreren Möglichkeiten dargestellt wird.

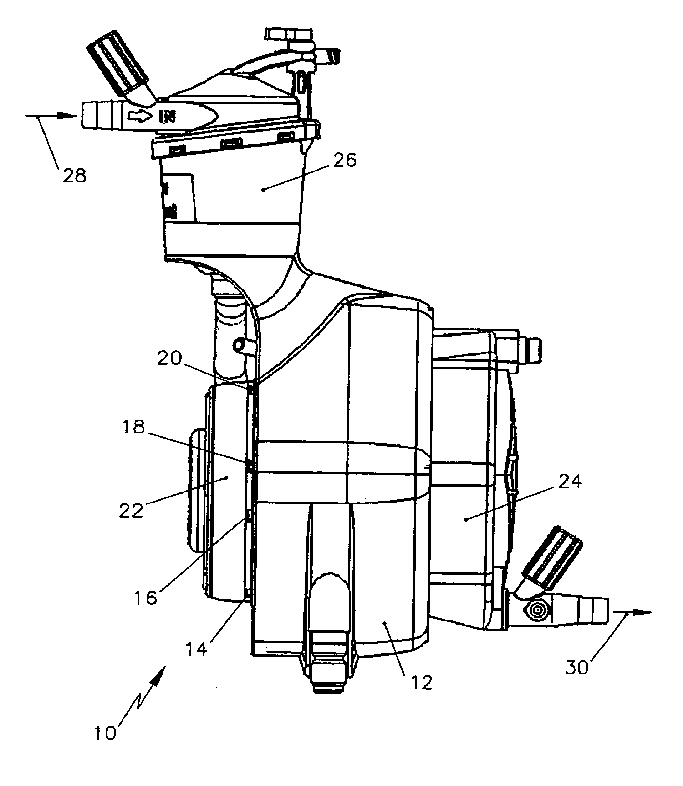

In der Figur ist beispielhaft mit 10 die erfindungsgemäße Vorrichtung in einer Seitenansicht gezeigt mit einem Oxygenator, in den ein Wärmetauscher integriert ist und die als eine Einheit anzusehende Gerätekombination ist als Bauteil 12 gekennzeichnet. Am Einlass in das Bauteil 12 sind Öffnungen 14, 16, 18, 20 vorgesehen, über die das aufzubereitende Blut, hier das venöse Blut, in das Bauteil 12 einströmen kann. Die Öffnungen 14, 16, 18, 20 sind bei Bedarf in unterschiedlichen Größen ausgebildet, sodass beispielsweise bevorzugt an der Öffnung 20 eine Entgasung des Blutstroms gefördert wird. Im Bereich der Öffnungen 14, 16, 18, 20 sind auch Verrastungsmittel bzw. Verbindungsmittel zwischen den Vorrichtungskomponenten vorgesehen.

In der Figur ist unmittelbar direkt an diese Öffnungen 14, 16, 18, 20 eine Pumpe, hier eine Zentrifugalpumpe, angekoppelt, sodass ohne Leitungsüberbrückung das aufzubereitende Blut über die Pumpe 22 in das Bauteil 12 gefördert werden kann. Die Pumpe 22 weist einen aufsteckbaren Antrieb auf, über den man die Zentrifugalpumpe so betreiben kann, dass der Antrieb nicht mit dem aufzubereitenden Blut in Berührung kommt (beispielsweise Magnetantrieb).

Die in der Figur gezeigten Öffnungen 14, 16, 18, 20 müssen nicht symmetrisch über den Einlass des Bauteils 12 verteilt sein. Denkbar ist auch eine einzige Öffnung, die einen Blutdurchflussquerschnitt von A ≥ 120 mm² zulässt. Die von den Öffnungen 14, 16, 18, 20 geschaffenen Blutdurchflussquerschnitte müssen in Summe ebenfalls einen Gesamtblutdurchflussquerschnitt von A ≥ 120 mm² ermöglichen.

Am Auslass des Bauteils 12 sind ebenfalls, in der Figur über die direkte Anflanschung an das Bauteil 12 verdeckt, mehrere Öffnungen ausgebildet, die ebenfalls einen Blutdurchflussquerschnitt von A ≥ 120 mm² anbieten. Die Einlassöffnungen in ein direkt an das Bauteil 12 angeflanschtes Blutfilter sind auf die Auslassöffnungen des Bauteils 12 angepasst, sodass das im Oxygenator/Wärmetauscher aufbereitete Blut möglichst schonend und unter geringster Belastung in das Blutfilter 24 einströmen kann.

Am Gehäuse des Bauteils 12 ist eine Blasenfalle 26 vorgesehen, die sicherstellt, dass in die Zentrifugalpumpe 22 ausschließlich blasenfreies Blut zur Aufbereitung in die Pumpe einströmt. Die Blasenfalle 26 ist mit der Pumpe 22 und die Pumpe 22 mit dem Bauteil 12 fluidtechnisch verbunden. Dabei sind zumindest zwischen der Pumpe 22 und dem Bauteil 12 keine Leitungen vorhanden. Die in der Figur gezeigten Bauteile sind unmittelbar an das Bauteil 12 angeflanscht.

In Pfeilrichtung 28 strömt venöses Blut in die Blasenfalle 26 ein und in Pfeilrichtung 30 strömt aus der erfindungsgemäßen Vorrichtung 10 arterielles Blut aus, das einem Patienten unmittelbar zugeführt werden kann.

Die gesamte Vorrichtung 10 ist in ein Gehäuse integrierbar, sodass erfindungsgemäß eine kompakte sichere Vorrichtung entsteht, die ein fehlerhaftes Betreiben nicht zulässt.

Bei einer Vorrichtung zur Behandlung von Blut in einem extrakorporalen Blutkreislauf, die mindestens einen Oxygenator, einen Wärmetauscher 12 und ein Blutfilter 24 aufweist, sind die Ein- und Auslässe in der Baueinheit Oxygenator/Wärmetauscher so ausgebildet, dass ein Blutdurchflussquerschnitt von A ≥ 120 mm², gewährleistet ist. Die erfindungsgemäße Vorrichtung 10 kann auch isoliert als ein einziges Bauteil 12 betrieben werden, das die Funktionen eines Oxygenators und eines Wärmetauschers mit einem Blutfilter übernimmt. Ist bei der Vorrichtung 10 eine Pumpe 22 vorgesehen, so kann deren Antrieb bei Bedarf abgenommen werden.

## Patentansprüche

1. Vorrichtung (10) zur Behandlung von Blut in einem extrakorporalen Blutkreislauf mit folgenden Vorrichtungskomponenten :
- einem Wärmetauscher,
- einem Oxygenator,
- einem Blutfilter (24),
wobei ein Einlass (14, 16, 18, 20) in und ein Auslass aus einer Baueinheit Wärmetauscher/Oxygenator vorgesehen ist, und wobei Ein- und Auslassquerschnitte der Vorrichtungskomponenten direkt zusammengekoppelt sind und der Wärmetauscher und der Oxygenator ein untrennbares Bauteil (12), **dadurch gekennzeichnet, dass** der Einlass (14, 16, 18, 20), der Auslass und im Übergangsbereich der Vorrichtungskomponenten zueinander die Blutdurchflussquerschnitte größer oder gleich 120 mm² ausgestaltet sind,
bilden dass der Wärmetauscher und der Oxygenator und eine einzige Einheit derart bilden, dass Wärmetauscherfasern in den Oxygenator integriert sind, wobei das Blutfilter (24) einen Blutdurchflussquerschnitt aufweist, der den Blutdurchflussquerschnitten der Baueinheit entspricht.

2. Vorrichtung (10) nach Anspruch 1, **dadurch gekennzeichnet, dass** am Einlass der Baueinheit eine Pumpe (22) vorgesehen ist, deren Auslass auf den Einlass der Baueinheit abgestimmt ist.

3. Vorrichtung (10) nach Anspruch 2, **dadurch gekennzeichnet, dass** als Pumpe eine Zentrifugalpumpe vorgesehen ist.

4. Vorrichtung nach Anspruch 2 oder 3, **dadurch gekennzeichnet, dass** die Vorrichtung (10) eine Blasenfalle (26) oder ein Blutreservoir aufweist, dass vor der Pumpe (22) vorgesehen ist und mit der Pumpe (22) fluidtechnisch verbunden ist.

## Claims

1. Device (10) for treating blood in an extracorporeal blood circuit with the following components:
- a heat exchanger,
- an oxygenator,
- a blood filter (24),
in which an inlet (14, 16, 18, 20) is provided into and an outlet is provided out of a heat exchanger/oxygenator assembly and in which the inlet and outlet cross sections of the components of the device are coupled together directly and the heat exchanger and the oxygenator form an inseparable assembly (12), **characterised in that** the inlet (14, 16, 18, 20), the outlet and the blood thoughput cross sections in the transition area between the components of the device are greater than or equal to 120 mm² and that the heat exchanger and the oxygenator form a single unit in such a way that heat exchanger fibres are incorporated in the oxygenator, in which the blood filter (24) has a blood throughput cross section, which corresponds to the blood throughput cross sections of the assembly.

2. Device (10) according to claim 1, **characterised in that** a pump (22) is provided at the inlet of the assembly, the outlet of which is adjusted to the inlet of the assembly.

3. Device (10) according to claim 2, **characterised in that** a centrifugal pump is provided as the pump.

4. Device according to claim 2 or 3, **characterised in that** the device (10) has a bubble trap (26) or a blood reservoir, which is provided before the pump (22) and is connected to the pump (22) by fluid technology.

## Revendications

1. Dispositif (10) de traitement du sang dans un circuit sanguin extracorporel, lequel dispositif a les composants suivants :
- un échangeur de chaleur,
- un oxygénateur,
- un filtre (24) pour le sang,
dans lequel il est prévu une entrée (14, 16, 18, 20) et une sortie dans une unité modulaire échangeur de chaleur/oxygénateur et
dans lequel des sections transversales d'entrée et de sortie sont directement accouplées aux composants du dispositif et l'échangeur de chaleur et l'oxygénateur forment une pièce inséparable (12), **caractérisé en ce que** l'entrée (14, 16, 18, 20), la sortie et, dans la zone de transition mutuelle des composants du dispositif, les sections transversales de passage du sang ont une surface supérieure ou égale à 120 mm² et forment une seule unité telle que les fibres de l'échangeur de chaleur soient intégrées à l'oxygénateur, dans lequel le filtre (24) pour le sang présente une section transversale de passage du sang qui correspond aux sections transversales de passage du sang de l'unité modulaire.

2. Dispositif (10) selon la revendication 1, **caractérisé en ce qu'**il est prévu à l'entrée de l'unité modulaire une pompe (22) dont la sortie est ajustée à l'entrée de l'unité modulaire.

3. Dispositif (10) selon la revendication 2, **caractérisé en ce qu'**il est prévu comme pompe une pompe centrifuge.

4. Dispositif selon la revendication 2 ou 3, **caractérisé en ce que** le dispositif (10) présente un piège à bulles (26) ou un réservoir de sang qui est prévu devant la pompe (22) et est raccordé à la pompe (22) par communication fluidique.
